(19) Europäisches Patentamt
European Patent Office
Office européen des brevets

(11) Veröffentlichungsnummer: **0 344 111**
**A2**

(12) # EUROPÄISCHE PATENTANMELDUNG

(21) Anmeldenummer: **89810365.0**

(22) Anmeldetag: **18.05.89**

(51) Int. Cl.4: **C 07 D 495/06**
C 07 D 517/06, C 07 F 7/08
//(C07D495/06,339:00,339:00),
(C07D517/06,345:00,345:00)

(30) Priorität: **27.05.88 CH 2008/88**
**19.07.88 CH 2752/88**

(43) Veröffentlichungstag der Anmeldung:
**29.11.89 Patentblatt 89/48**

(84) Benannte Vertragsstaaten:
**CH DE FR GB IT LI NL**

(71) Anmelder: **CIBA-GEIGY AG**
**Klybeckstrasse 141**
**CH-4002 Basel (CH)**

(72) Erfinder: **Baumann, Marcus, Dr.**
**Arlesheimerstrasse 18**
**CH-4053 Basel (CH)**

**Fischer, Walter, Dr.**
**Vogesenstrasse 77**
**CH-4153 Reinach (CH)**

**Kvita, Vratislav, Dr.**
**Vogesenstrasse 71**
**CH-4153 Reinach (CH)**

**Mayer, Carl W., Dr.**
**Steingrubenweg 224**
**CH-4125 Riehen (CH)**

**Wernet, Wolfgang, Dr.**
**Schillerstrasse 40**
**D-7800 Freiburg (DE)**

(54) **Substituierte Tetrathio- und Tetraselenotetracene.**

(57) Substituierte Tetrathio- und Tetraselenotetracene der Formel I

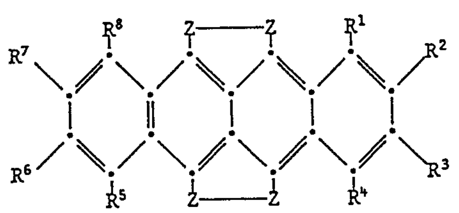

(I)

worin Z für -S- oder -Se- steht und

a) $R^1$, $R^2$, $R^3$ und $R^4$ H sind, und $R^5$ bis $R^8$ je für H stehen und mindestens einer der Reste $R^5$ bis $R^8$ unabhängig voneinander einen Substituenten aus der Gruppe unsubstituiertes oder mit Halogen, -CN, $-CONR^9R^{10}$, $-OR^9$, $-SR^9$ oder $-COOR^9$ substituiertes $C_1$-$C_{20}$-Alkyl$(X)_p$ mit Ausnahme von $C_1$-$C_4$-Alkyl und Methoxy, $C_2$-$C_{18}$-Alkenyl$(X)_p$, $C_2$-$C_{18}$-Alkinyl$(X)_p$, $C_3$-$C_8$-Cycloalkyl$(X)_p$, ($C_1$-$C_{12}$-Alkyl)-$C_3$-$C_8$-cycloalkyl$(X)_p$, $C_3$-$C_8$-Cycloalkyl-$C_rH_{2r}(X)_p$, ($C_1$-$C_{12}$-Alkyl)-$C_3$-$C_8$-cycloalkyl-$C_rH_{2r}(X)_p$, Phenyl$(X)_p$, ($C_1$-$C_{12}$-Alkyl)phenyl$(X)_p$, Phenyl-$C_rH_{2r}(X)_p$, ($C_1$-$C_{12}$-Alkyl)phenyl-$C_r$-$H_{2r}(X)_p$ darstellen, r für 1 oder 2 und p für 0 oder 1 stehen und X -O-, -S-, -SO-oder $-SO_2$-bedeutet, oder $R^5$ bis $R^8$ unabhängig einen Substituenten aus der Gruppe -Br, $-CF_3$, -CN, -Si($C_1$-$C_4$-Alkyl)$_3$, $-S(C_mH_{2m}O)_n$ $R^{11}$ oder $-O(C_mH_{2m}O)_n$ $R^{11}$ bedeuten, oder je eines von $R^5$ bis $R^8$ für -F oder -Cl und mindestens ein weiteres von $R^5$ bis $R^8$ einen Substituenten der zuvor definierten Gruppen einschliesslich $C_1$-$C_4$-Alkyl und Methoxy bedeutet,
$R^9$ und $R^{10}$ unabhängig voneinander H, $C_1$-$C_{12}$-Alkyl, Phenyl oder $(C_mH_{2m}O)_q$ $R^{11}$, oder $R^9$ und $R^{10}$ zusammen Tetramethylen, Pentamethylen, 3-Oxapentylen oder $-CH_2CH_2NR^9CH_2CH_2$- bedeuten,
$R^{11}$ für H oder $C_1$-$C_{12}$-Alkyl steht,
m für eine Zahl von 2 bis 4, n für eine Zahl von 2 bis 20 und q für eine Zahl von 1 bis 20 stehen, oder

b) $R^1$ bis $R^8$ unabhängig voneinander H oder einen der zuvor definierten Substituenten einschliesslich $C_1$-$C_4$-Alkyl und Methoxy darstellen, oder $-COOR^9$ oder $-CONR^9R^{10}$ oder je zwei benachbarte Reste von $R^1$ bis $R^8$ -CO-O-CO- oder $-CO-NR^9-CO-$ bedeuten, und mindestens eines von $R^1$ bis $R^4$ sowie $R^5$ bis $R^8$ einen Substituenten bedeuten, mit Ausnahme von $R^2$, $R^3$, $R^6$ und $R^7$ gleich -F

EP 0 344 111 A2

und Methyl, und wobei $R^9$ und $R^{10}$ die zuvor angegebenen Bedeutungen haben.

Sie bilden mit Elektronenacceptoren Charge-Transferkomplexe, die als organische elektrische Leiter, Displays, optische Schalter und Sensoren verwendet werden können.

**Beschreibung**

## Substituierte Tetrathio- und Tetraselenotetracene

Die Erfindung betrifft substituierte Tetrathio- und Tetraselenotetracene und ein Verfahren zu deren Herstellung.

Es ist bekannt, dass Mono- und Difluortetrathio- und -tetraselenotetracene und Tetrathio- und Tetraselenotetracenmono- und -dicarbonsäuren, -säureester und -säureamide mit Elektronenacceptoren elektrisch leitende Charge-Transferkomplexe bilden, siehe z.B. US-A 4 522 754, EP-A- 0 153 905, US-A-4 617 151 und DE-A-3 635 124.

In CA 103:225177h (1985) wird beschrieben, dass man Kristalle von Di(tetrathiotetracen)-triiodid mit Methoxytetrathiotetracen und ICl dotieren kann. Isopropyltetrathiotetracen ist in CA 86: 189773n (1977) beschrieben. Tertiärbutyltetrathiotetracen ist als Bestandteil eines Kation-Radikalsalzes in CA 99: 175630b (1983) beschrieben. Die Herstellung dieser substituierten chalkogenierten Tetracene ist mit aufwendigen Synthesen verbunden.

Ein Gegenstand der Erfindung sind Verbindungen der Formel I

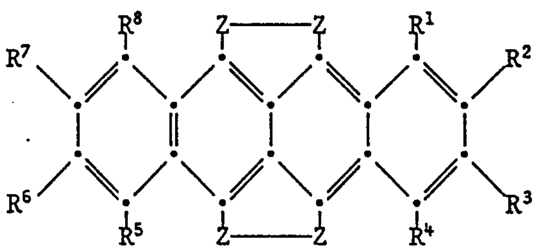 (I),

worin Z für -S- oder -Se- steht und

a) $R^1$, $R^2$, $R^3$ und $R^4$ H sind,
und $R^5$ bis $R^8$ je für H stehen und mindestens einer der Reste $R^5$ bis $R^8$ unabhängig voneinander einen Substituenten aus der Gruppe unsubstituiertes oder mit Halogen, -CN, $-CONR^9R^{10}$, $-OR^9$, $-SR^9$ oder $-COOR^9$ substituiertes $C_1$-$C_{20}$-Alkyl$-(X)_p$ mit Ausnahme von $C_1$-$C_4$-Alkyl und Methoxy, $C_2$-$C_{18}$-Alkenyl$-(X)_p$, $C_2$-$C_{18}$-Alkinyl$-(X)_p$, $C_3$-$C_8$-Cycloalkyl$-(X)_p$, ($C_1$-$C_{12}$-Alkyl)-$C_3$-$C_8$-cycloalkyl$-(X)_p$, $C_3$-$C_8$-Cycloalkyl-$C_rH_{2r}-(X)_p$, ($C_1$-$C_{12}$-Alkyl)-$C_3$-$C_8$-cycloalkyl-$C_rH_{2r}-(X)_p$, Phenyl$-(X)_p$, ($C_1$-$C_{12}$-Alkyl)phenyl$-(X)_p$, Phenyl-$C_rH_{2r}-(X)_p$, ($C_1$-$C_{12}$-Alkyl)phenyl-$C_rH_{2r}-(X)_p$ darstellen,
r für 1 oder 2 und p für 0 oder 1 stehen und X -O-, -S-, -SO-oder $-SO_2-$ bedeutet,
oder $R^5$ bis $R^8$ unabhängig einen Substituenten aus der Gruppe -Br, $-CF_3$, -CN, $-Si(C_1\text{-}C_4\text{-Alkyl})_3$, $-S-(C_mH_{2m}O)_n-R^{11}$ oder $-O-(C_mH_{2m}O)_n-R^{11}$ bedeuten,
oder je eines von $R^5$ bis $R^8$ für -F oder -Cl und mindestens ein weiteres von $R^5$ bis $R^8$ einen Substituenten der zuvor definierten Gruppen einschliesslich $C_1$-$C_4$-Alkyl und Methoxy bedeutet,
$R^9$ und $R^{10}$ unabhängig voneinander H, $C_1$-$C_{12}$-Alkyl, Phenyl oder $-(C_mH_{2m}O)_q-R^{11}$, oder $R^9$ und $R^{10}$ zusammen Tetramethylen, Pentamethylen, 3-Oxapentylen oder $-CH_2CH_2NR^9CH_2CH_2-$ bedeuten,
$R^{11}$ für H oder $C_1$-$C_{12}$-Alkyl steht,
m für eine Zahl von 2 bis 4, n für eine Zahl von 2 bis 20 und q für eine Zahl von 1 bis 20 stehen, oder

b) $R^1$ bis $R^8$ unabhängig voneinander H oder einen der zuvor definierten Substituenten einschliesslich $C_1$-$C_4$-Alkyl und Methoxy darstellen, oder $-COOR^9$ oder $-CONR^9R^{10}$ oder je zwei benachbarte Reste von $R^1$ bis $R^8$ -CO-O-CO- oder $-CO-NR^9-CO-$ bedeuten, und mindestens eines von $R^1$ bis $R^4$ sowie $R^5$ bis $R^8$ einen Substituenten bedeuten, mit Ausnahme von $R^2$, $R^3$, $R^6$ und $R^7$ gleich -F und Methyl, und wobei $R^9$ und $R^{10}$ die zuvor angegebenen Bedeutungen haben.

Bedeuten im Rahmen der vorangehenden Definitionen $R^1$ bis $R^8$ $C_1$-$C_{20}$-Alkyl$-(X)_p$, so kann die Alkylgruppe linear oder verzweigt sein und vorzugsweise 1 bis 18, besonders 1 bis 12 und insbesondere 1 bis 6 C-Atome enthalten. Beispiele für Alkylgruppen sind Methyl, Ethyl und die Isomeren von Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl und Eicosyl. X steht bevorzugt für -O-, -S- und $-SO_2-$ und p bevorzugt für 1.

Bedeuten im Rahmen der vorangehenden Definitionen $R^1$ bis $R^8$ $C_2$-$C_{18}$-Alkenyl$-(X)_p$ so kann die Alkenylgruppe linear oder verzweigt sein und vorzugsweise 3 bis 12, besonders 3 bis 6 C-Atome enthalten. Das Alkenyl enthält bevorzugt terminale Doppelbindungen. Einige Beispiele sind Ethenyl, Allyl, Prop-1-en-1- oder -2-yl, But-1-en-1- oder -2- oder -3- oder -4-yl, But-2-en-1- oder -2-yl, Pent-1-en- oder Pent-2-en-1- oder -2- oder -3- oder -4- oder -5-yl, Hex-1-en- oder Hex-2-en- oder Hex-3-en-1- oder -2- oder -3- oder -4- oder -5- oder -6-yl, Heptenyl, Octenyl, Nonenyl, Decenyl, Undecenyl, Dodecenyl, Tetracenyl, Hexadecenyl und Octadecenyl. X steht bevorzugt für -O-, -S- und $-SO_2-$ und p bevorzugt für 1.

Bedeuten im Rahmen der vorangehenden Definition $R^1$ bis $R^8$ $C_2$-$C_{18}$-Alkinyl$-(X)_p$, so kann die Alkinylgruppe linear oder verzweigt sein und vorzugsweise 3 bis 12, besonders 3 bis 6 C-Atome enthalten. Die Dreifachbindung befindet sich vorzugsweise in terminaler Stellung. Einige Beispiele sind Ethinyl, Propargyl, But-1-in-3- oder -4-yl, But-2-in-1-yl, Pent-1-in-3- oder -4- oder -5-yl, Hex-1-in-3- oder -4- oder -5- oder -6-yl, Hex-2-in-1- oder -4- oder -5- oder -6-yl, Hex-3-in-1- oder -2-yl, Heptinyl, Octinyl, Noninyl, Decinyl, Undecinyl

und Dodecinyl. X steht bevorzugt für -O-, -S- und $-SO_2-$ und p bevorzugt für 1.

Bedeuten $R^1$ bis $R^8$ im Rahmen der vorangehenden Definitionen $C_3$-$C_8$-Cycloalkyl$-(X)_p$, so kann es sich um Cyclopropyl, Cyclobutyl, Cyclopentyl, Cyclohexyl, Cycloheptyl oder Cyclooctyl handeln. X steht bevorzugt für -O-, -S- oder $-SO_2-$. Bevorzugt ist $C_3$-$C_6$-Cycloalkyl und p ist bevorzugt 1.

Bedeuten $R^1$ bis $R^8$ im Rahmen der vorangehenden Definitionen $C_3$-$C_8$-Cycloalkyl-$C_rH_{2r}-(X)_p$, ($C_1$-$C_{12}$-Alkyl)-$C_3$-$C_8$-cycloalkyl$-(X)_p$ oder ($C_1$-$C_{12}$-Alkyl)-$C_3$-$C_8$-cycloalkyl-$C_rH_{2r}-(X)_p$, so kann die Alkylgruppe linear oder verzweigt sein und vorzugsweise 1 bis 6, besonders 1 bis 4 C-Atome enthalten. In der $-C_rH_{2r}$-Gruppe steht r bevorzugt für 1. X steht bevorzugt für -O-, -S- oder $-SO_2-$. Die Cycloalkylgruppe stellt in diesen Substituenten bevorzugt $C_3$-$C_6$-Cycloalkyl dar und p ist bevorzugt 1.

Bedeuten $R^1$ bis $R^8$ im Rahmen der vorangehenden Definitionen ($C_1$-$C_{12}$-Alkyl)phenyl$-(X)_p$, Phenyl-$C_rH_{2r}-(X)_p$ oder ($C_1$-$C_{12}$-Alkyl)phenyl$-(X)_p$, so kann die Alkylgruppe linear oder verzweigt sein und vorzugsweise 1 bis 6, besonders 1 bis 4 C-Atome enthalten. In der $-C_rH_{2r}$-Gruppe steht r bevorzugt für 1, X ist bevorzugt -O-, -S- oder $-SO_2-$ und p ist bevorzugt 1.

$R^1$ bis $R^8$ können im Rahmen der vorangehenden Definitionen unsubstituiert oder ein- oder mehrfach, bevorzugt ein- bis dreifach, insbesondere ein-oder zweifach substituiert sein. Ist der Substituent Halogen, so handelt es sich bevorzugt um -F, -Cl oder -Br. Ist der Substituent $-CONR^9R^{10}$, so bedeuten $R^9$ und $R^{10}$ bevorzugt unabhängig voneinander H oder $C_1$-$C_4$-Alkyl und besonders Methyl oder Ethyl. Ist der Substituent $-OR^9$, $-SR^9$ oder $-COOR^9$, so stellt $R^9$ bevorzugt H, $C_1$-$C_4$-Alkyl oder $-(C_mH_{2m}O)_q$ $R^{11}$ dar, worin $R^{11}$ H oder $C_1$-$C_4$-Alkyl bedeutet und m für 2 oder 3 und q für 1 bis 12 stehen.

Sind $R^1$ bis $R^8$ im Rahmen der vorangehenden Definitionen Halogen, so handelt es sich um -F, -Cl oder -Br, besonders -F und -Cl.

Sind $R^1$ bis $R^8$ im Rahmen der vorangehenden Definitionen $-COOR^9$ oder $-CONR^9R^{10}$, so sind $R^9$ und $R^{10}$ unabhängig voneinander bevorzugt $C_1$-$C_6$-Alkyl oder $R^9$ und $R^{10}$ zusammen bevorzugt Tetra- oder Pentamethylen oder 3-Oxapentylen.

Sind $R^1$ bis $R^8$ im Rahmen der vorangehenden Definitionen $-Si(C_1$-$C_4$-Alkyl$)_3$, so handelt es sich bevorzugt um $-Si(C_1$- oder $C_2$-Alkyl) und insbesondere um Trimethylsilyl.

Sind $R^1$ bis $R^8$ im Rahmen der vorangehenden Definitionen $-S-(C_mH_{2m}-O)_n$ $R^{11}$ oder $-O-(C_mH_{2m}-O)_n$ $R^{11}$, so stellt $R^{11}$ bevorzugt H oder $C_1$-$C_4$-Alkyl dar, m steht bevorzugt für 2 oder 3 und n bevorzugt für 1 bis 12, besonders 1 bis 6.

Benachbarte Reste für die Gruppen -CO-O-CO- und $-CO-NR^9-CO-$ sind besonders $R^2$ und $R^3$ und/oder $R^6$ und $R^7$.

$R^9$ und $R^{10}$ enthalten als Alkyl bevorzugt 1 bis 6, besonders 1 bis 4 C-Atome und sind insbesondere Methyl oder Ethyl. Das Alkyl kann linear oder verzweigt sein.

Bedeuten $R^9$ und $R^{10}$ die Gruppe $-(C_mH_{2m}-O)_qR^{11}$, so stellen bevorzugt m 2 oder 3, q 1 bis 12, besonders 2 bis 6 und $R^{11}$ H oder $C_1$-$C_4$-Alkyl dar.

In einer bevorzugten Ausführungsform stehen $R^9$ und $R^{10}$ unabhängig voneinander für H, $C_1$-$C_4$-Alkyl, Phenyl, oder $R^9$ und $R^{10}$ zusammen für Tetramethylen, Pentamethylen oder 3-Oxapentylen.

In einer bevorzugten Ausführungsform steht p für 1 und in einer weiteren bevorzugten Ausführungsform stehen $R^1$, $R^4$, $R^5$ und $R^8$ für H.

In einer anderen bevorzugten Ausführungsform stellen $R^6$ oder $R^7$, oder $R^6$ und $R^7$ einen Substituenten dar und $R^1$ bis $R^5$ und $R^8$ bedeuten H. Ebenfalls bevorzugt ist eine Ausführungsform, in der $R^6$ oder $R^6$ und $R^7$ sowie $R^2$ oder $R^2$ und $R^3$ einen Substituenten darstellen und $R^1$, $R^4$, $R^5$ und $R^8$ H bedeuten.

Eine bevorzugte Ausführungsform von Verbindungen der Formel I sind solche worin $R^1$ bis $R^4$ H sind, und $R^5$ bis $R^8$ je für H stehen und mindestens einer der Reste $R^5$ bis $R^8$ unabhängig voneinander einen Substituenten aus der Gruppe unsubstituiertes oder mit -F, -Cl, -CN, $-CONR^9R^{10}$, $-OR^9$, $-SR^9$ oder $-COOR^9$ substituiertes $C_1$-$C_{18}$-Alkyl$-(X)_p$, $C_3$-$C_{12}$-Alkenyl$-(X)_p$, $C_3$-$C_{12}$-Alkinyl$-(X)_p$, $C_5$- oder $C_6$-Cycloalkyl$-(X)_p$, ($C_1$-$C_6$-Alkyl)-$C_5$- oder $C_6$-cycloalkyl$-(X)_p$, $C_5$- oder $C_6$-Cycloalkyl-$CH_2-(X)_p$, ($C_1$-$C_6$-Alkyl)-$C_5$- oder $C_6$-cycloalkyl-$CH_2-(X)_p$, Phenyl$-(X)_p$, ($C_1$-$C_6$-Alkyl) phenyl$-(X)_p$, Benzyl$-(X)_p$ oder ($C_1$-$C_6$-Alkyl)benzyl$-(X)_p$ darstellen;

X -O-, -S-, -SO- oder $-SO_2-$ und p 0 oder 1 bedeuten;

oder $R^5$ bis $R^8$ unabhängig einen Substituenten aus der Gruppe $-CF_3$, -CN, $-Si(C_1$- oder $C_2$-Alkyl$)_3$, $-S-(C_mH_{2m}-O)_n$ $R^{11}$, oder $-O-(C_mH_{2m}-O)_p$ $R^{11}$;

oder je eines von $R^5$ bis $R^8$ für -F oder -Cl und mindestens ein weiteres von $R^5$ bis $R^8$ einen Substituenten der zuvor definierten Gruppen einschliesslich $C_1$-$C_4$-Alkyl und Methoxy darstellen;

$R^9$ und $R^{10}$ unabhängig voneinander H, $C_1$-$C_6$-Alkyl, $-CH_2CH_2OH$, oder $R^9$ und $R^{10}$ zusammen Tetramethylen, Pentamethylen oder $-CH_2CH_2NR^9CH_2CH_2-$ bedeuten, $R^{11}$ für H oder $C_1$-$C_4$-Alkyl steht; und m 2 oder 3 und n eine Zahl von 2 bis 12 darstellen.

Insbesondere bevorzugt sind solche Verbindungen der Formel I, worin $R^1$ bis $R^5$ und $R^8$ H sind und $R^6$ ein Substituent und $R^7$ H oder $R^6$ und $R^7$ ein Substituent aus der Gruppe $-Si(CH_3)_3$; $-CF_3$; -CN; mit $-COOR^9$ substituiertes $C_1$-$C_6$-Alkyl oder Benzyl; unsubstituiertes oder mit -OH substituiertes $C_1$-$C_{18}$-Alkoxy oder $C_1$-$C_{18}$-Alkylthio; unsubstituiertes oder mit -F, -Cl, -OH, $C_1$-$C_4$-Alkoxy, $C_1$-$C_4$-Alkylthio substituiertes Phenyl-X-, Benzyl-X-, $C_1$-$C_4$-Alkylphenyl-X- oder $C_1$-$C_4$-Alkylbenzyl-X- mit X gleich -O-, -S- oder $-SO_2-$; oder unsubstituiertes $C_3$-$C_{12}$-Alkenyloxy darstellen.

Eine andere bevorzugte Ausführungsform sind solche Verbindungen der Formel I, worin $R^1$, $R^4$, $R^5$ und $R^8$ H sind, und mindestens eines von $R^2$ und $R^3$ sowie von $R^6$ und $R^7$ einen Substituenten aus der Gruppe

$-COOR^9$, $-CONR^9R^{10}$, unsubstituiertes oder mit -F, -Cl, -CN, $-CONR^9R^{10}$, $-OR^9$, $-SR^9$ oder $-COOR^9$ substituiertes $C_1$-$C_{18}$-Alkyl$(X)_p$, $C_3$-$C_{12}$-Alkenyl$(X)_p$, $C_3$-$C_{12}$-Alkinyl$(X)_p$, $C_5$- oder $C_6$-Cycloalkyl$(X)_p$, ($C_1$-$C_6$-Alkyl)-$C_5$- oder $C_6$-cycloalkyl$(X)_p$, $C_5$- oder $C_6$-Cycloalkyl-$CH_2(X)_p$, ($C_1$-$C_6$-Alkyl)-$C_5$- oder $C_6$-cycloalkyl-$CH_2(X)_p$, Phenyl$(X)_p$, ($C_1$-$C_6$-Alkyl)phenyl$(X)_p$, Benzyl$(X)_p$ oder ($C_1$-$C_6$-Alkyl)benzyl$(X)_p$ darstellen;
X -O-, -S-, -SO- oder $-SO_2-$ und p 0 oder 1 bedeuten; oder aus der Gruppe $-CF_3$, -CN, -Si($C_1$- oder $C_2$-Alkyl)$_3$, $-S(C_mH_{2m}-O)_n R^{11}$, oder $-O(C_mH_{2m}-O)_p R^{11}$; oder
$R^2$ und $R^3$ zusammen -CO-O-CO- oder $-CO-NR^9-CO-$ sind und mindestens eines von $R^6$ und $R^7$ einen der zuvor definierten Substituenten darstellen oder gemeinsam -CO-O-CO- oder $-CO-NR^9-CO-$ sind;
$R^9$ und $R^{10}$ unabhängig voneinander H, $C_1$-$C_6$-Alkyl, $-CH_2CH_2OH$, oder $R^9$ und $R^{10}$ zusammen Tetramethylen, Pentamethylen oder $-CH_2CH_2NR^9CH_2CH_2-$ bedeuten; und m 2 oder 3 und n eine Zahl von 2 bis 12 darstellen.
Insbesondere bevorzugt hierbei sind solche Verbindungen der Formel I, worin der Substituent ausgewählt ist aus der Gruppe $-COOR^9$, $-Si(CH_3)_3$; $-CF_3$; -CN; mit $-COOR^9$ substituiertes $C_1$-$C_6$-Alkyl oder Benzyl; unsubstituiertes oder mit -OH substituiertes $C_1$-$C_{18}$-Alkoxy oder $C_1$-$C_{18}$-Alkylthio; unsubstituiertes oder mit -F, -Cl, -OH, $C_1$-$C_4$-Alkoxy, $C_1$-$C_4$-Alkylthio substituiertes Phenyl-X-, Benzyl-X-, $C_1$-$C_4$-Alkylphenyl-X- oder $C_1$-$C_4$-Alkylbenzyl-X- mit X gleich -O-, -S- oder $-SO_2-$; oder unsubstituiertes $C_3$-$C_{12}$-Alkenyloxy darstellen. Besonders ausgewählte Substituenten sind hierbei $-CF_3$, $C_1$-$C_{18}$-Alkoxy und $-COOR^9$.

Die Herstellung von Verbindungen der Formel I kann nach bekannten oder einem neuen Verfahren erfolgen. Zentrale Zwischenprodukte sind hierbei entsprechend substituierte Naphthacen-5,12-dione der Formel III

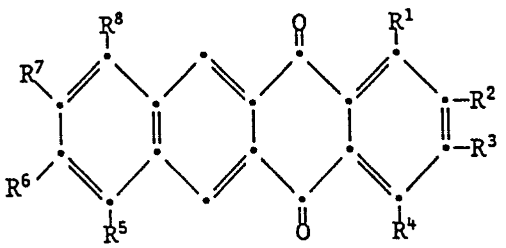

(III),

die teilweise bekannt sind oder z.B. nach folgenden Verfahren hergestellt werden können.

Die Verbindungen der Formel III können hergestellt werden, indem man ein Naphthalindicarbonsäureanhydrid der Formel IV

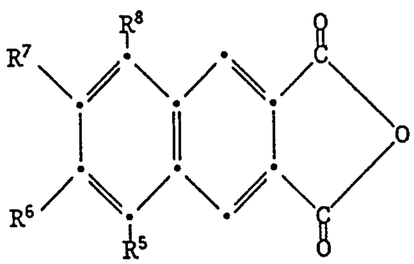

(IV)

in Gegenwart einer Lewissäure mit einem Benzol der Formel V

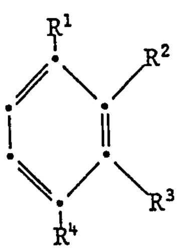

(V)

umsetzt, wobei $R^1$ bis $R^8$ die zuvor angegebenen Bedeutungen haben, und gegebenenfalls Verbindungen der Formel III, worin mindestens eines von $R^1$ bis $R^8$ $-NO_2$ oder Halogen bedeuten, mit einer nukleophilen Verbindung substituiert. Halogen ist bevorzugt -Br, -Cl und besonders -F. Für die nukleophile Substitution geeignete Verbindungen sind besonders solche der Formel ($R^1$ bis $R^8$)-X-H, worin X -O-, -S-, -SO- oder $-SO_2-$ sind, Malonsäureester oder -nitrile und Phenylessigsäurenitril. Die Verbindungen können in Form ihrer Alkalisalze, z.B. Li-, Na- oder K-Salze verwendet werden. Ebenso ist es möglich, die nukleophile Substitution in Gegenwart von Basen, wie z.B. Alkalilaugen oder -carbonaten durchzuführen.

Die Verbindungen der Formel III können auch hergestellt werden, indem man in einer Diels-Alder-Reaktion eine Verbindung der Formel VI

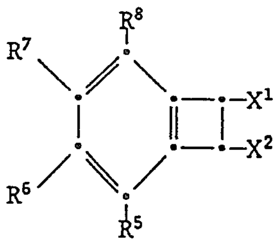

(VI)

worin $R^5$, $R^6$, $R^7$ und $R^8$ die zuvor angegebenen Bedeutungen haben und zusätzlich $-NO_2$ sein können, und $X^1$ und $X^2$ unabhängig voneinander für -Cl, -Br - oder -I stehen, unter Abspaltung von $HX^1$ und $HX^2$ mit einer Verbindung der Formel VII

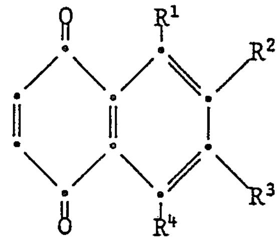

(VII),

worin $R^1$, $R^2$, $R^3$ und $R^4$ die zuvor angegebenen Bedeutungen haben, und zusätzlich $-NO_2$ sein können, umsetzt. In den Substituenten $R^1$ bis $R^8$ ist p bevorzugt 1.

Diese Reaktion wird vorteilhaft bei Temperaturen von 50 bis 250°C, bevorzugt 80 bis 200°C durchgeführt. Zweckmässig wird ein inertes Lösungsmittel verwendet, zum Beispiel polare aprotische Lösungsmittel. Einige Beispiele sind aromatische Kohlenwasserstoffe (Benzol, Toluol, Xylol, Chlorbenzol und Dichlorbenzol), Nitrile (Acetonitril), Ether (Dibutylether, Dioxan, Tetrahydrofuran, Ethylenglykol- oder Diethylenglykoldimethylether). Die Isolierung und Reinigung kann nach üblichen Methoden erfolgen, z.B. Kristallisation, Sublimation oder Chromatographie.

Verbindungen der Formel VI sind teilweise bekannt (siehe z.B. H.P. Cava et al., J. Am. Chem. Soc., S. 1701 (1957) und J.W. Barton et al., J. Chem. Soc. Perkin Trans. 1, S. 967-971 (1986)), bzw. nach analogen Verfahren herstellbar. Die für die Herstellung benötigten substituierten 1,2-Bis(dichlor- oder -dibrommethyl)benzole sind ebenfalls teilweise bekannt oder durch übliche elektrophile oder nukleophile Substitutionsreaktionen entsprechender o-Dimethylbenzole, und deren anschliessenden Chlorierung oder Bromierung mit z.B. N-Chlor- oder N-Bromsuccinimid erhältlich.

Die 1,4-Naphthochinone der Formel VII sind bekannt und z.B. durch nukleophile Substitution von gegebenenfalls geschützten und substituierten Halogen- oder Nitro-1,4-naphthochinonen mit z.B. den zuvor beschriebenen Verbindungen in Gegenwart von Alkalimetallverbindungen ($K_2CO_3$, $Cs_2CO_3$, KOH, NaOH, $NaNH_2$, $NaOCH_3$, $NaOC_2H_5$) oder mit Alkalimetall-Verbindungen z.B. von Li, K, Na, Rb oder Cs, erhältlich. Halogen-und Nitro-naphthochinone sind z.B. in Houben-Weyl, Chinone I, Band 7/3b (1977) beschrieben. Die Naphthochinone der Formel VII können auch in bekannter Weise durch elektrophile oder nukleophile Substitution von gegebenenfalls substituierten Naphthalinen, Dihydro- oder Tetrahydronaphthalinen und anschliessende Umwandlung in die substituierten 1,4-Naphthochinone hergestellt werden.

Die Verbindungen der Formel III können auch hergestellt werden, indem man 1,2-Bis(dihalogenmethyl)benzole der Formel

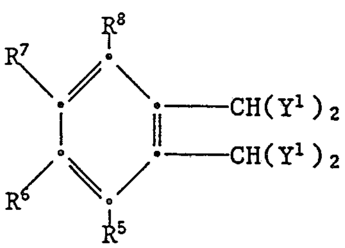

,

worin $Y^1$ für Cl, Br oder I steht, in Gegenwart von NaI in einem organischen Lösungsmittel mit einer Verbindung der Formel VII umsetzt. Diese Methode ist von J.W. McOmie in Synthesis, S. 416-417 (1973) beschrieben.

Verbindungen der Formel I können auch erhalten werden, indem man Anthracen-1,4-chinone der Formel

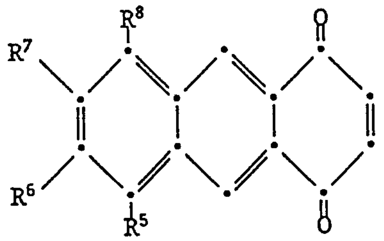

mit einem α-Pyron der Formel VIII

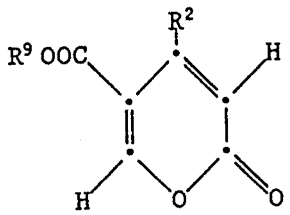

(VIII),

oder einem Butadien der Formel IX

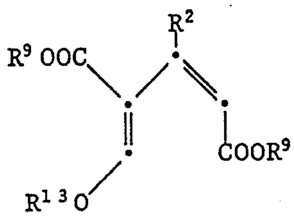

(IX)

umsetzt, wobei $R^{13}$ $C_1$-$C_6$-Alkyl ist, $R^9$ die zuvor angegebene Bedeutung hat und bevorzugt $C_1$-$C_6$-Alkyl ist. Diese Methode und die Herstellung von α-Pyronen ist in der US-PS 4,617,151 und derEP-A-0 195 743 beschrieben.

Verbindungen der Formeln VIII und IX sind z.B. folgendermassen erhältlich, wobei $X^1$ ein Alkalimetall bedeutet:

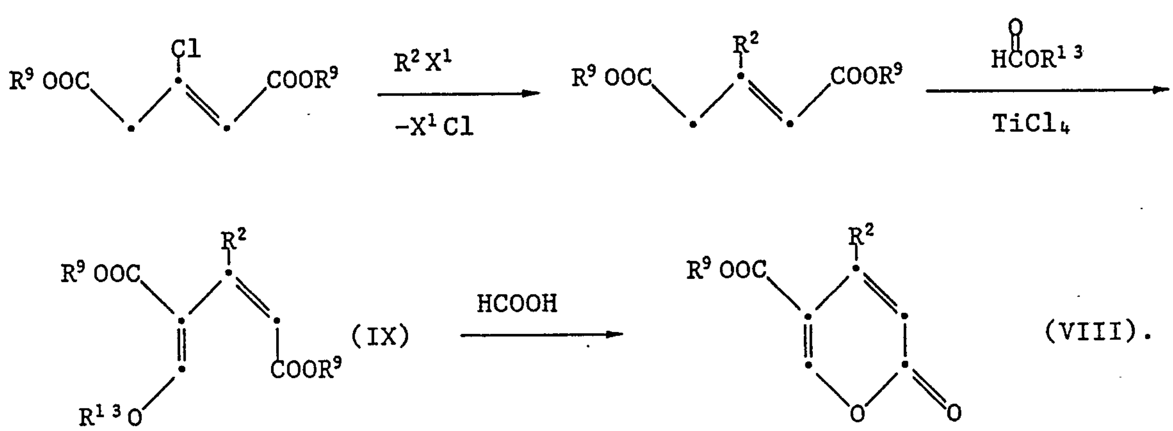


(IX) (VIII).

Wenn $R^1$ bis $R^8$ einen Polyoxaalkylenrest bedeuten, so erhält man solche Verbindungen auch durch Umsetzung von Verbindungen der Formel I mit $R^1$ bis $R^8$ gleich Hydroxyalkoxy mit Epoxiden. Ferner ist es möglich die Reste $R^1$ bis $R^8$ durch klassische Reaktionen abzuwandeln, wie z.B. Hydrolyse, Ver- oder Umesterung, Amidierung, Oxidation, Reduktion oder Dehydrierung. Carbonsäureester können in bekannter Weise mit $HF/SF_4$ in die Trifluormethylderivate umgewandelt werden.

Die Verbindungen der Formel I sind auf verschiedenen Reaktionswegen aus den Naphthacendionen der Formel III erhältlich.

In einem neuen Verfahren werden aus den Naphthacendionen der Formel III in an sich bekannter Weise [vergleiche T. Kametani in Chem. Pharm. Bull. 26(12), S. 3820-3825 (1978)] durch reduktive Acylierung 5,12-Diacyloxynaphthacene der Formel X erhalten,

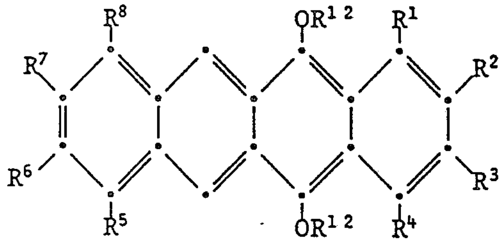

(X),

worin $R^1$ bis $R^8$ z.B. unabhängig voneinander H und mindestens eines von $R^1$ bis $R^8$ ein Substituent aus der Gruppe -F, -Si($C_1$-$C_4$-Alkyl)$_3$, -$COOR^{10}$; oder je zwei benachbarte Reste von $R^1$ bis $R^8$ -CO-O-CO-; oder mindestens eines von $R^1$ bis $R^8$ unsubstituiertes oder mit -F, -OH, $C_1$-$C_{12}$-Alkoxy, $C_1$-$C_{12}$-Acyloxy oder -$COOR^{10}$ substituiertes $C_1$-$C_{20}$-Alkyl$\leftarrow X \rightarrow_p$, $C_3$-$C_8$-Cycloalkyl$\leftarrow X \rightarrow_p$, $C_1$-$C_{12}$-Alkyl-$C_3$-$C_8$-cycloalkyl$-\leftarrow X \rightarrow_p$, $C_3$-$C_8$-Cycloalkyl-$CH_2$$\leftarrow X \rightarrow_p$, $C_1$-$C_{12}$-Alkyl-$C_3$-$C_8$-cycloalkyl-$CH_2$$\leftarrow X \rightarrow_p$, $C_6$-$C_{10}$-Aryl-X-, $C_7$-$C_{20}$-Alkaryl-X-, $C_7$-$C_{12}$-Aralkyl$\leftarrow X \rightarrow_p$ oder $C_8$-$C_{20}$-Alkaralkyl$\leftarrow X \rightarrow_p$ oder -Y$\leftarrow C_mH_{2m}$-O$\rightarrow_n$ $R^{10}$ darstellen;
$R^{10}$ für H oder $C_1$-$C_{18}$-Alkyl steht, X -O-, -S-, -SO- oder -$SO_2$- und Y -O-oder -S- bedeuten, und p für 0 oder 1, m für eine Zahl von 2 bis 6 und n für eine Zahl von 2 bis 20 stehen;
und $R^{12}$ unsubstituiertes oder mit -F substituiertes $C_1$-$C_4$-Acyl, besonders Acetyl darstellt.

Die Verbindungen der Formel X können direkt mit Schwefel in einem Lösungsmittel, z.B. 1,2,4-Trichlorbenzol, und in Gegenwart katalytischer Mengen einer Sulfonsäure, z.B. p-Toluolsulfonsäure, bei Temperaturen zwischen 100 bis 300°C zu den entsprechenden Verbindungen der Formel I umgesetzt werden.

Die Naphthacendione der Formel III können in an sich bekannter Weise [siehe Ch. Marschalk, Bull. Soc. Chim. France 427 (1948) und S. 931 und 1122 (1939 sowie EP-A-0 153 905] zu entsprechend substituierten 5,12-Dihydrotetracen oder Tetracen reduziert und mit Schwefel direkt zu den entsprechenden Tetrathiotetracenen umgesetzt werden.

Die Tetracene können in bekannter Weise (siehe EP-A-0 153 905) zu Verbindungen der Formel II chloriert werden,

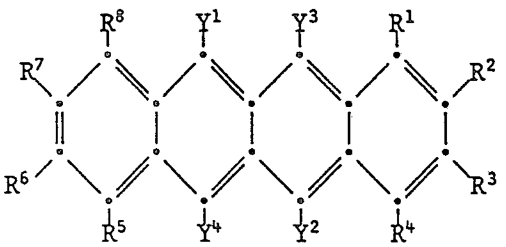

(II),

worin $R^1$ bis $R^8$ die zuvor angegebenen Bedeutungen haben, $Y^1$ und $Y^2$ -Cl und $Y^3$ und $Y^4$ H bedeuten oder $Y^3$ und $Y^4$ -Cl und $Y^1$ und $Y^2$ H bedeuten. In einem allgemeinen Herstellungsverfahren für die Verbindungen der Formel I können die Verbindungen der Formel II in bekannter Weise mit Schwefel, Selen, einem Alkalimetallsulfid oder -selenid umgesetzt werden.

Bei allen Zwischenstufen ist es möglich, die Reste $R^1$ bis $R^8$ nach klassischen, zuvor erwähnten Reaktionen abzuwandeln.

Bei den Verbindungen der Formel I handelt es sich um überwiegend kristalline und gefärbte Verbindungen, deren Eigenschaften durch den Substitutionsgrad und die Art der Substituenten gezielt beeinflusst werden kann: z.B. das Oxidationspotential; oder die Lage des Absorptionsmaximums, das in den längerwelligen Bereich verschoben werden kann, was für laser-optische Anwendungen vorteilhaft ist.

Aus den Verbindungen der Formel I können mit Elektronenacceptoren elektrisch leitende Charge-Transferkomplexe (CT-Komplexe) hergestellt werden. Mit deren funktionellen Substituenten kann man sie an Polymere binden, z.B. als Seitengruppen in Polymere einbauen (vgl. US-PS 4,617,151). Die CT-Komplexe eignen sich auch zur Herstellung von z.B. von antistatischen Beschichtungen photographischer Filmelemente, Magnetbänder, elektrophotographischer Filmelemente und elektronischer Komponenten (siehe US-PS 3,634,336). Die chalkogenierten Tetracene weisen ferner elektrochrome Eigenschaften auf; sie können für elektrochrome Displays verwendet werden. Auf Grund ihres langwelligen $\lambda_{max}$ eignen sich einige auch als laser-optische Datenspeicher [Nach. Chem. Techn. Lab. 35, S. 255 ff (1987)]. Sie können auch als Anodenmaterial in organischen Solid-State-Batterien (EP-A-0 090 598) eingesetzt werden. CT-Komplexe von substituierten Tetrathio- oder Tetraselenotetracenen können zur Erzielung antistatischer Eigenschaften auch in thermoplastische, duroplastische oder elastomere Polymere eingearbeitet werden. Hierzu werden zweckmässig z.B. die substituierten Tetrathio- bzw. Tetraselenotetracene zusammen mit einem löslichen Polymer oder einer Vorstufe davon und einem Elektronenacceptor, z.B. einem Halogen bildenden Mittel (organische halogenierte Verbindungen wie z.B. Bromoform, Trichlorbrommethan, Tetrabrommethan, Hexachlorpropan, Perchlorbutadien, 1,3-oder 1,4-Dichlor-2-buten, 1,4-Bis-(trichloromethyl)-benzol, Iodacetonitril, Iodoform, Tetrachlorethylen, Perchlorcyclobutadien, N-Chlor-, -brom oder -iodsuccinimid) gegebenenfalls zusammen mit einem weiteren inerten Lösungsmittel gelöst und das überschüssige Halogen bildende

Mittel und das Lösungsmittel bei höherer Temperatur verdampft. Die gebildete Zusammensetzung enthält im Polymer ein Netzwerk nadelförmiger Kristalle des CT-Komplexes, wenn das chal kogenierte Tetracen unsubstituiert ist oder kleine Substituenten (z.B. F, $CH_3$, $CF_3$) enthält. Solche Zusammensetzungen weisen eine hohe elektrische Leitfähigkeit auf. Diese kann noch verbessert werden, wenn man ein substituiertes Tetrathio-oder Tetraselenotetracen der Formel I mitverwendet, das kein solches Netzwerk bildet und das in der Polymermatrix in feiner Verteilung vorliegt, da solche substituierten Tetrathio- oder Tetraselenotetracene keine oder nur eine geringe Kristallisationstendenz im Polymer besitzen.

Die nachfolgenden Beispiele erläutern die Erfindung näher.

A) Herstellung von Ausgangsprodukten

Beispiele a-g:

10,35 mMol 2-substituiertes Naphthacen-5,12-dion, 40 ml Essigsäureethylester, 25 ml Acetanhydrid und 31,05 mMol Kaliumacetat werden unter Rühren mit 31,05 mMol Zinkpulver versetzt. Nach 40 Minuten Rühren bei 25°C wird das Reaktionsgemisch filtriert und der Rückstand viermal mit $CH_2Cl_2$ gewaschen. Die Filtrate werden eingedampft und der Rückstand aus $CH_2Cl_2$/Pentan und dann aus Toluol umkristallisiert. Ausbeuten und Schmelzpunkte der erhaltenen 2-substitierten 5,12-Diacetoxynaphthacene sind in Tabelle 1 angegeben.

Tabelle 1

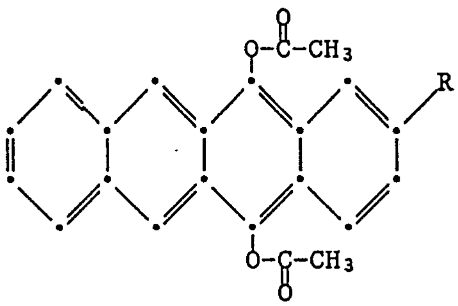

| Beispiel Nr. | R | Ausbeute (%) | Schmelzpunkt (°C) |
|---|---|---|---|
| a | $-OCH_2CH_3$ | 79 | 150-153 |
| b | $-OCH_2-CH(C_2H_5)(CH_2)_3CH_3$ | 71 | 130-135 |
| c | $-O-n-C_8H_{17}$ | 84 | 107-111 |
| d | $-O-n-C_{18}H_{37}$ | 49 | 155-157 |
| e | $-SCH_2CH_3$ | 77 | 130-135 |
| f | $-C(CH_3)(COOC_2H_5)_2$ | 63 | 230 (Zersetzung) |
| g | $-CF_3$ | 91 | >250 |

Die entsprechenden 2-substituierten Naphthacen-5,12-dione der Beispiele a-f sind durch nukleophile Substitution von 2-Fluornaphtacen-5,12-dion erhältlich.

Beispiel h: 2-(Trifluormethyl-)-naphthacen-5,12-dion

5,65 g (25 mMol) 6-(Trifluormethyl-)-1,4-naphthochinon, 9,82 g (ca 37 mMol) 1,2-Dibrombenzocyclobuten (verunreinigt mit etwas 2-Brom-1-iodbenzocyclobuten) und 100 ml Xylol werden während 16 Stunden mit einem Wasserabscheider am Rückfluss gehalten. Das Gemisch wird abgekühlt und der Niederschlag abfiltriert umd mit Xylol gewaschen. Ausbeute 5,82 g (71 %); Smp. 252-254°C.

Analog wird bei der Herstellung des im nachfolgenden Beispiel i verwendeten 2,3-Bis-(trifluormethyl-)-naphthacen-5,12-dions (Ausbeute 59 %; Smp. >280°C) verfahren.

Beispiel i:

1,65 mMol 2,3-Bis-(trifluormethyl)-naphthacen-5,12-dion, 5 ml Essigsäureethylester, 4,96 mMol Kaliumacetat und 3 ml Acetanhydrid werden unter Zugabe von 0,1 g Pd/C (5 %) bei 20-25°C während 35 Minuten hydriert. Das Gemisch wird filtriert und der Rückstand dreimal mit $CH_2Cl_2$ ausgewaschen. Die Filtrate werden eingedampft und der Rückstand aus $CH_2Cl_2$/Pentan umkristallisiert. Ausbeute 76 % 2,3-Bis-(trifluormethyl)-5,12-diacetoxy-naphthacen; Schmelzpunkt > 250°.

Beispiele j-m: 2-(2'-Hydroxyethoxy)-naphthacen-5,12-dion

27,6 g (0,1 mol) 2-Fluor-5,12-naphthacendion, 32,5 g (0,1 mol) $Cs_2CO_3$ und 300 ml Ethylenglycol werden in einem Sulfierkolben unter Stickstoff vorgelegt. Nach Erwärmen auf 125°C wird $3\frac{1}{4}$ h gerührt. Danach wird das Reaktionsgemisch in 3000 ml salzsaures Wasser gegossen, das ausgefallene Produkt abfiltriert und mehrmals mit Wasser gewaschen. Nach dem Trocknen im Vakuum bei 80°C werden 30,3 g (97,1 %) reines Produkt erhalten, Smp. 208,8°C. Analog wird in den Beispielen k-m verfahren (s. Tabelle 2).

Beispiel n: 2-(3'-Butenoxy)-naphthacen-5,12-dion

10 g (0,036 mol) 2-Fluor-5,12-naphthacendion, 26 g (0,028 mol) $Cs_2CO_3$ und 10,41 g (0,14 mol) 3-Butenol werden in 200 ml DMF unter Stickstoff vorgelegt. Die Reaktionsmischung wird auf 125°C erwärmt und $4\frac{1}{2}$ h gerührt. Nach dem Fällen in 4000 ml salzsaurem Wasser wird abfilteriert (Rohausbeute 94 %). Nach Chromatographie an Silicagel ($CH_2Cl_2$) werden 63 % reines Produkt erhalten; Smp. 149°C.

Tabelle 2

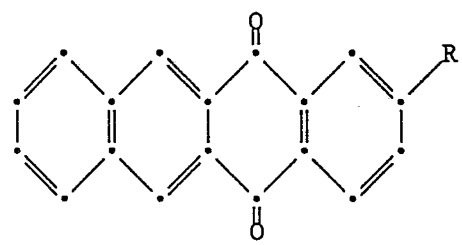

| Beispiel | R | Smp. (°C) | Reaktionszeit (Stunden) | Ausbeute (%) | Lösungsmittel | Reaktionstemperatur (°C) |
|---|---|---|---|---|---|---|
| j | $O-(CH_2)_2-OH$ | 208 | 3 1/4 | 97 | i.S. | 125 |
| k | $O-(CH_2)_4-OH$ | 190 | 7 1/2 | 70 | i.S. | 120 |
| l | $O-(CH_2)_6-OH$ | 153 | 1 1/4 | 70 | DMSO | 120 |
| m | $O-(CH_2)_{10}-OH$ | 113 | 3/4 | 73 | i.S. | 125 |
| n | $O-(CH_2)_2-CH=CH_2$ | 149 | 4 1/2 | 63 | DMF | 125 |

i.S.: Alkohol ist gleich Lösungsmittel

DMF : Dimethylformamid

Beispiel o: 2-Cyano-naphthacen-5,12-dion

10 g (33,2 mMol) Naphthacen-5,12-dion-2-carbonsäureamid, 10,18 g (66,4 mMol) $POCl_3$ und 200 ml DMF werden bei 10°C, dann bei 25°C während 2 Stunden gerührt. Das Gemisch wird auf Eiswasser ausgetragen. Der Niederschlag wird abfiltriert, dreimal mit Wasser gewaschen und getrocknet. Aus o-Dichlorbenzol umkristallisiert, werden 7,35 g (78 %) Rohprodukt erhalten.
IR-Spektrum (KBr): 1678 $cm^{-1}$: Chinon; 2240 $cm^{-1}$: CN
Massenspektrum: M/e = 283 ($M^+$) (Basispeak); 255; 227; 226; 100.

Beispiel p:

Eine Mischung aus 0,1 Mol 4-Trimethylsilyl-1,2-bis-dibromomethylbenzol, 0,12-0,2 Mol substituiertem Naphthochinon, 0,6 Mol Natriumiodid und 1000 ml Aceton bzw. Acetonitril wird unter Rühren und $N_2$-Atmosphäre während 5 Stunden am Rückfluss gekocht. Nach dem Abkühlen wird der ausgefallene Niederschlag abfiltriert und mit Wasser digeriert. Das auf diese Weise erhaltene Filtrat wird zur Trockne

verdampft, der Rückstand in $CH_2Cl_2$ gelöst und mit $NaHSO_3$ gewaschen und getrocknet. Anschliessend wird der Rückstand einer Wasserdampfdestillation unterworfen und die nichtflüchtigen Teile mit Ether extrahiert, getrocknet und dann sublimiert. Man erhält in 79 % Ausbeute 8-Trimethylsilylnaphthacen-5,12-dion mit einem Smp. von 168-170°C.

Auf analoge Weise wird bei direkter Sublimation des Filtrats 2,3-Trifluormethyl-8-methoxy-naphthacen-5,12-dion in 33 % Ausbeute erhalten, Smp. > 280°C.

Beispiel q:

Man verfährt wie in Beispiel p, indem man Aceton und entsprechend substituierte Bis-dibrommethylbenzole und Naphthochinone verwendet und das Filtrat direkt sublimiert. Man erhält eine 1:1-Mischung von 8- und 9-Methoxynaphthacen-5,12-dion-2-carbonsäuremethylester in 50 % Ausbeute, Smp. 246-248°C.

Beispiel r

Die Naphthacen-5,12-dione der Beispiele j-p werden nach den folgenden Methoden zu den entsprechenden Tetracenen reduziert.

Methode A

30 mMol Pyridin werden in 25 ml Eisessig gelöst und am Rückfluss gerührt. 10 mMol 2-R-Naphthacen-5,12-dion und 60 mMol Zinkstaub werden separat vermischt und in die obige Lösung unter Rühren langsam eingetragen. Nach 15 Minuten Rühren am Rückfluss wird das Gemisch auf Salzsäure/Wasser ausgetragen und der Niederschlag unter Abdekantieren der Hauptmenge an Zink filtriert. Der Rückstand wird dreimal mit Wasser gewaschen, getrocknet und aus o-Dichlorbenzol unter Heissfiltration umkristallisiert.

Methode B

1,53 mol Aluminiumspäne werden in 1000 ml Butanol in einem Sulfierkolben, ausgestattet mit Gaseinlass, Rückflusskühler und Rührer, vorgelegt. Die Reaktionsmischung wird auf Rückfluss erhitzt. Nach einer Induktionsperiode von 5 bis 15 Minuten beginnt eine stark exotherme Reaktion. Die Heizung wird entfernt und mit einem Eis/Wasserbad vertauscht. Die Innentemperatur sollte 100°C nicht unterschreiten. Nach ca. 10 Minuten ist die Reaktion weitgehend beendet. Die Lösung wird weitere 30 Minuten auf Rückfluss gehalten, dann in ein anderes Reaktionsgefäss filtriert. Danach werden 0,06 mol 5,12-Naphthacendion zugegeben und unter Stickstoff und Rühren 12 Stunden am Rückfluss erhitzt. Danach wird die Reaktionsmischung in 3000 ml 15 % HCl gegossen und mit Ethanol homogenisiert. Das ausgefallene Produkt wird abfiltriert, mit Methanol und Wasser gewaschen und bei 70°C im Vakuum getrocknet. Die weitere Reinigung erfolgt durch Umkristallisation aus Chlorbenzol.

Tabelle 3

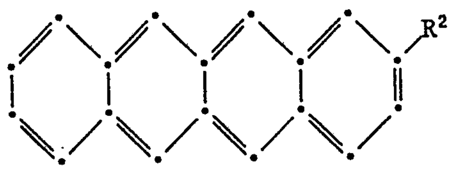

| $R^2$ | Methode | Ausbeute (%) | Smp. (°C) |
|---|---|---|---|
| $-CN$ | A | 32 | > 250 |
| $-O(CH_2)_2-OH$ | B | 73 | 316 |
| $-O(CH_2)_4-OH$ | B | 70 | 293 |
| $-O(CH_2)_6-OH$ | B | 46 | 271 |
| $-O(CH_2)_{10}-OH$ | B | 60 | 254 |
| $-O(CH_2)_2-CH=CH_2$ | B | 69 | 275 |
| $-Si(CH_3)_3$ | A | 13 | > 260 |

Beispiel s: Reduktion zu Dihydrotetracenen

Eine Mischung aus 0,08 Mol entsprechend substituiertem Naphthacen-5,12-dion, 0,24 Mol Pyridin, 0,48 Mol Zink und 600 ml Eisessig werden unter Rühren und $N_2$-Atmosphäre 4 Stunden am Rückfluss gekocht. Anschliessend werden nochmals 0,24 Mol Zink zugegeben und 16 Stunden am Rückfluss gekocht. Nun wird die Reaktionslösung heiss filtriert und das Filtrat im Vakuum zur Trockne verdampft. Der Rückstand wird zwischen $CH_2Cl_2$ und Wasser verteilt und die organische Phase mit $NaHCO_3$-Lösung und HCl 1N gewaschen. Nach dem Verdampfen des Lösungsmittels wird der Rückstand mit $CH_2Cl_2$ flash-chromatographiert (vgl.Tabelle 4).

Tabelle 4

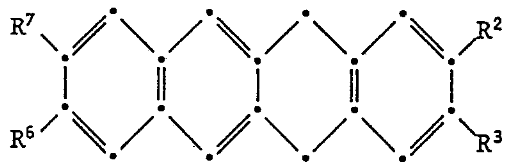

| $R^2$ | $R^3$ | $R^6$ | $R^7$ | Ausbeute (%) | Smp. (°C) |
|---|---|---|---|---|---|
| $-COOCH_3$ | H | $-OCH_3$ a) | H | 57 | 162-165 |
| $-CF_3$ | $-CF_3$ | $-OCH_3$ | H | 66 | Oel |
| $-CF_3$ | $-CF_3$ | $-COOCH_3$ | $COOCH_3$ | 30 | 186-188 |

a) 1:1-Mischung der 8- und 9-Stellungsisomeren

Beispiel t: Dehydrierung zu Tetracen

Eine Mischung aus 4 mMol substituiertes Dihydronaphthacen, 4mMol Chloranil und 70 Eisessig wird 1-4 Stunden unter $N_2$-Atmosphäre und Rühren am Rückfluss erhitzt. Nach dem Abkühlen werden die gebildeten Kristalle abgetrennt und mit $CH_2Cl_2$ und Methanol gewaschen (siehe Tabelle 5a).

Tabelle 5a

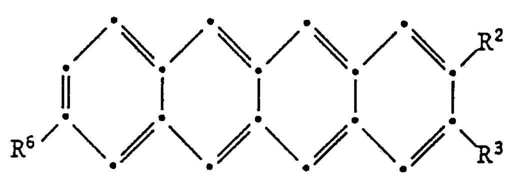

| $R^2$ | $R^3$ | $R^6$ | Ausbeute (%) | Smp. (°C) |
|---|---|---|---|---|
| $-COOCH_3$ | H | $-OCH_3$ a) | 95 | > 280 |
| $-CF_3$ | $-CF_3$ | $-OCH_3$ | 27 | 167-168 |

Auf analoge Weise werden die in Tabelle 5b erwähnten Tetracene hergestellt.

Tabelle 5b

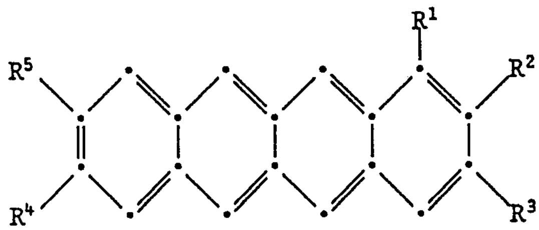

| $R^1$ | $R^2$ | $R^3$ | $R^4$ | $R^5$ | $\lambda_{max}$ (nm) |
|---|---|---|---|---|---|
| | -CO-O-CO | | $-OCH_3$ | | 446 |
| | $-COOC_2H_5$ | $-COOC_2H_5$ | $-OCH_3$ | | 496 |
| | $-CONH_2$ | | $-OCH_3$ | | 493 |
| $-CF_3$ | $-COOCH_3$ | | | $-OCH_3$ | 512 |
| $-CF_3$ | $-COOCH_3$ | | $-OCH_3$ | | 503 |
| | $-CF_3$ | $-CF_3$ | $-CF_3$ | $-CF_3$ | 467 |
| | $-CF_3$ | $-CF_3$ | $-CF_3$ | | 472 |
| | -CN | | $-OCH_3$ | | 503 |
| | $-CONH_2$ | | $-OCH_3$ | | 493 |

Beispiel u: 2,3-Dicyanotetracen

Eine Mischung aus 3,5 g Anthracen-2,3-dialdehyd (E. Mallonli et al., Synthesis 1980, S. 689), 1,8 g Bernsteinsäuredinitril, 3 g $K_2CO_3$ und 250 ml Dimethylsulfoxid werden unter Rühren 30 Minuten auf 60-70°C erwärmt. Anschliessend wird auf 15°C abgekühlt und mit 50 ml Wasser versetzt. Der ausgefallene Niederschlag wird abfiltriert und bei 300°C sublimiert. Ausbeute 485 mg (12 %), $\lambda_{max}$: 772 nm.

B) Herstellungsbeispiele:

Beispiele 1-6:

In einem 250 ml Kolben mit Rückflusskühler und Gaseinleitungsrohr werden unter leichtem Argonfluss 1,83 mMol substituiertes Naphthacen-5,12-diacetat, 15,8 mVal $S_8$ und 0,026 mMol p-Toluolsulfonsäure in 100 ml 1,2,4-Trichlorbenzol 5 1/2 Stunden am Rückfluss erhitzt. Danach wird die dunkelgrüne Lösung am Hochvakuum eingedampft.

Das Rohprodukt wird über eine Kieselgelflashsäule (240 g Kieselgel, Ø 7 cm) mit $CCl_4$ chromatographiert. [Das Kieselgel muss zuvor mit $CCl_4$, das 2 % Triethylamin enthält, behandelt und darauf mit reinem $CCl_4$ gewaschen werden, bis das Eluat wieder neutral ist.] Die dunkelgrün gefärbten Fraktionen enthalten das gereinigte 2-substituierte 5,6,11,12-Tetrathiotetracen. Spektrale Daten und Ausbeuten sind in Tabelle 6 angegeben.

Tabelle 6

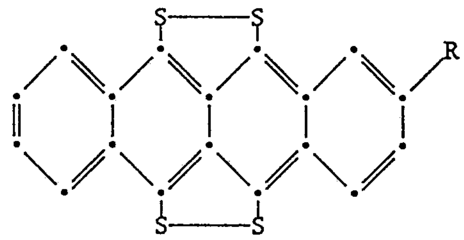

| Beispiel Nr. | R | Massenspektrum ($M^+$) | $\lambda_{max}$ (nm) in 1,2,4-Trichlorbenzol | Ausbeute (%) |
|---|---|---|---|---|
| 1 | $-OC_2H_5$ | 396 | 700 | 24[a)] |
| 2 | $-O-n-C_8H_{17}$ | 480 | 699 | 50[b)] |
| 3 | $-OCH_2CH(C_2H_5)(CH_2)_3CH_3$ | 480 | 698 | 8[a)] |
| 4 | $-O-n-C_{18}H_{37}$ | 620 | 698 | 50[a)] |
| 5 | $-C(CH_3)(COOC_2H_5)_2$ | 524 | 704 | 10[a)] |
| 6 | $-SC_2H_5$ | 412 | 711 | 3 % |

a) sublimiert

b) chromatographiert

Beispiel 7:

In einem 100 ml Kölbchen mit Rückflusskühler und Gaseinlassrohr werden unter leichtem Argonfluss 251 mg (0,61 mMol) 2-Trifluormethylnaphthacen-5,12-diacetat, 78 mg (2,43 mVal) $S_8$ und 2 mg (0,01 mMol) p-Toluolsulfonsäure in 35 ml 1,2,4-Trichlorbenzol 20 Stunden am Rückfluss erhitzt. Nach dem Abkühlen wird am Hochvakuum (HV) das Lösungsmittel abgedampft, der Rückstand mit Hexan ausgekocht, das schwarze Pulver abfiltriert und am HV bei 60°C getrocknet. Man erhält 203 mg (79 %) Rohprodukt.

Dieses wird bei 190°C (1,3 x $10^{-4}$ mbar) sublimiert, wobei 67,5 mg (35,6 %) reines 2-Trifluormethyl--5,6-11,12-tetrathiotetracen erhalten werden (schwarze Nädelchen) . Massenspektrum: $M^+ = 420$. $\lambda_{max}$ (1,2,4-Trichlorbenzol): 725, 665, 484 nm.

Beispiel 8: 2,3-Trifluormethyl-5,6,11,12-tetrathiotetracen

Es wird wie in Beispiel 37 verfahren und 2,3-Trifluormethylnaphtacen-5,12-diacetat verwendet. Ausbeute: 75,6 mg (30 %) nach Sublimation. Massenspektrum: $M^+ = 488$. $\lambda_{max}$ (1,2,4-Trichlorbenzol): 755 nm.

Beispiel 9:

In einem 50 ml Kölbchen mit Rückflusskühler und Gaseinleitungsrohr werden unter leichtem Argonfluss 60 mg (0,2 mMol) 2-Trimethylsilyltetracen, 40 mg (12,5 mMol) Schwefel in 30 ml 1,2,4-Trichlorbenzol während 18 Stunden refluxiert. Nach Abkühlen der nunmehr tiefgrünen Lösung wird zur Trockne eingedampft. Der schwarze kristalline Festkörper wird mit 4 x 20 ml Hexan gewaschen. Ausbeute an 2-Trimethylsilyl-5,6,11,12-tetrathiotetracen: 72 mg (85 %). Massenspektrum: $M^+ = 424$. Die Fragmentierung steht im Einklang mit der erwarteten Stuktur. $\lambda_{max}$ (Trichlorbenzol): 712, 653, 478 nm.

Beispiele 10 und 11:

Unter Verwendung entsprechend substituierter Tetracene wird wie in Beispiel 9 verfahren (siehe Tabelle 7a).

Tabelle 7a

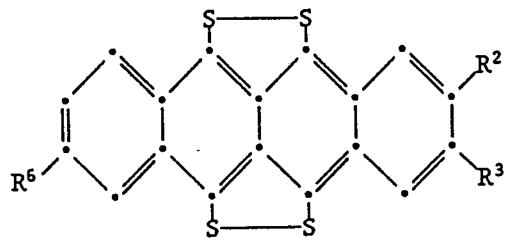

| Beispiel | $R^2$ | $R^3$ | $R^6$ | Ausbeute | $\lambda_{max}$ (Trichlorbenzol) |
|---|---|---|---|---|---|
| 10 | $-COOCH_3$ | H | $-OCH_3$ a) | 85%(roh) | 738/743 |
| 11 | $-CF_3$ | $-CF_3$ | $-OCH_3$ | | 753 |

Analog Beispiel 9 werden die Tetrathiotetracene gemäss Tabelle 7b hergestellt.

Tabelle 7b

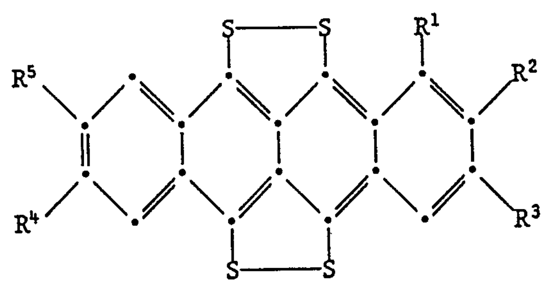

| $R^1$ | $R^2$ | $R^3$ | $R^4$ | $R^5$ | $\lambda_{max}$ (nm) |
|---|---|---|---|---|---|
| $-CF_3$ | | $-COOCH_3$ | | $-OCH_3$ | 772 |
| $-CF_3$ | | $-COOCH_3$ | $-OCH_3$ | | 775 |
| | $-CN$ | $-CN$ | | | 805 |
| | $-CF_3$ | $-CF_3$ | $-CF_3$ | $-CF_3$ | 768 |
| | $-COOCH_3$ | $-COOCH_3$ | $-CF_3$ | $-CF_3$ | 768 |
| | $-CF_3$ | $-CF_3$ | $-CF_3$ | | 754 |
| | -CO-O-CO- | | $-OCH_3$ | | 776 |
| | $-COOC_2H_5$ | $-COOC_2H_5$ | $-OCH_3$ | | 763 |
| | $-CN$ | | $-OCH_3$ | | 750 |
| | $-CONH_2$ | | $-OCH_3$ | | 738 |

Beispiele 12-17:

3,16 mMol 2-R-Naphthacen, 37 mMol $S_8$ und 30 mg (92 E-6 mol) Cäsiumcarbonat werden in 30 ml Dimethylsulfoxid (DMSO) unter Argon vorgelegt. Danach wird auf 120°C erwärmt und 5 1/2 Stunden gerührt. Die Lösung wird in Wasser eingebracht und filtriert. Nach Umkristallisation aus Chlorbenzol wird das Produkt isoliert (siehe Tabelle 8).

Tabelle 8

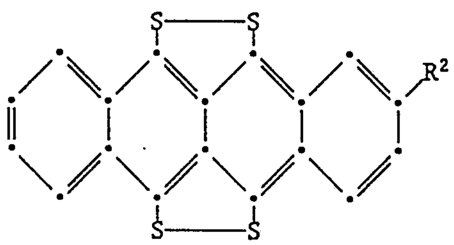

| Beispiel | $R^2$ | Ausbeute (%) | $\lambda_{max}$ (nm) |
|---|---|---|---|
| 12 | $-O(CH_2)_2OH$ | 45 | 696[b)] |
| 13 | $-O(CH_2)_4OH$ | 65 | 691[b)] |
| 14 | $-O(CH_2)_6OH$ | 58 | 697[b)] |
| 15 | $-O(CH_2)_{10}OH$ | 64 | 696[b)] |
| 16 | $-O(CH_2)_2-CH=CH_2$ | 65 | 689[c)] |
| 17 | $-OCH_2C(CH_2OH)_2$ mit $CH_2-CH_3$ am C | 73 | 694[b)] |

b) DMSO

c) Dimethylformamid (DMF)

Beispiel 18:

In einem 50 ml-Dreihalskölbchen mit Gaseinleitungsrohr und Rückflusskühler werden 1 mMol 2-Cyanotetracen in 20 ml Nitrobenzol (Fluka puriss, über A4 Molekularsieb getrocknet) unter leichtem Argonfluss vorgelegt und auf 5°C abgekühlt. Dazu werden 297 mg 2,2 mMol) Sulfurylchlorid (puriss Fluka) in 5 ml Nitrobenzol zugetropft, sodass die Temperatur nicht über 7°C steigt. Es wird darnach 1 Stunde bei 5°C gerührt, wobei ein beiger Niederschlag entsteht. Darnach wird auf Raumtemperatur aufwärmen gelassen und anschliessend für 2 Stunden bei 90°C gehalten. Nach Abkühlen auf Raumtemperatur wird das Reaktionsgemisch am Hochvakuum eingedampft und der rostbraune Festkörper im Temperaturgradienten bei 170°C Ofentemperatur sublimiert. Ausbeute 134 mg (42 %) rote Nädelchen. Smp. 225-230°; Massenspektrum: $M^+ = 321$ (2Cl).

In einem 25 ml Dreihalskölbchen mit Gaseinleitungsrohr und Rückflusskühler werden 0,081 mMol 2-Cyano-dichlortetracen, 0,0324 mMol $Se_8$ in 5 ml Dowtherm A 25 Stunden zum Rückfluss erhitzt. Dabei nimmt die Reaktionslösung eine intensiv dunkel-grüne Farbe an. Nach Abkühlen auf Raumtemperatur wird filtriert und der getrocknete schwarze Festkörper im Hochvakuum ($10^{-7}$ Torr) bei 280°C sublimiert. Ausbeute 5 mg (~ 11 %) schwarze Nädelchen.

$\lambda_{max}$ (1,2,4-Trichlorbenzol): 765, 700, 493 nm

Massenspektrum: $M^+ = 566$ (4Se). Die Fragmentierung steht mit der erwarteten Struktur von 2-Cyano-5,6,11,12-tetraselenotetracen im Einklang.

Beispiele 19 und 21:

Es wird analog wie in Beispiel 18 verfahren unter Verwendung von 2,3-Dicyano-, 2-Trifluormethyl- bzw. 2,3-Di(trifluormethyl)tetracen (siehe Tabelle 9).

Tabelle 9

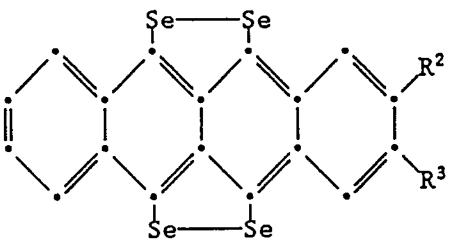

| Beispiel | $R^2$ | $R^3$ | Ausbeute (sublimiert, %) | $\lambda_{max}$ (Trichlorbenzol) |
|---|---|---|---|---|
| 19 | $-CF_3$ | H | 8,5 | 735 |
| 20 | $-CF_3$ | $-CF_3$ | 5 | 760 |
| 21 | -CN | -CN | 57 | 820 |

C) Anwendungsbeispiel: Elektrochromie

In eine Elektrochromiezelle, bestehend aus einer Teflonmembran und in je 0,5 mm Abstand einer Anode und Kathode aus ITO-Glas, werden zur Anodenseite 1 mg 2,3-Di(trifluormethyl)-5,6,11,12-tetrathioetracen und 100 mg $LiClO_4$, gelöst in 5 ml Aceton, und zur Kathodenseite eine Lösung von 1 mg 2,3-Di(trifluormethyl)-5,6,11,12-tetrathiotetracen-perchlorat (CT-Komplex) und 100 mg $LiClO_4$ in 5 ml Aceton eingefüllt. Nach Anlegen einer Spannung von 2 Volt wechseln innerhalb weniger Sekunden die Farben von grün nach rotviolett auf der Anodenseite und von rotviolett nach grün auf der Kathodenseite. Durch Umpolung der Spannung werden in beiden Zellenhälften die ursprünglichen Farben erhalten. Der gleiche Effekt wird beobachtet, wenn man Nitrobenzol oder Dimethylformamid als Lösungsmittel verwendet.

**Patentansprüche**

1. Verbindungen der Formel I

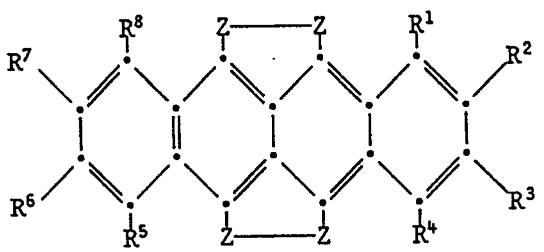

(I)

worin Z für -S- oder -Se- steht und

a) $R^1$, $R^2$, $R^3$ und $R^4$ H sind,

und $R^5$ bis $R^8$ je für H stehen und mindestens einer der Reste $R^5$ bis $R^8$ unabhängig voneinander einen Substituenten aus der Gruppe unsubstituiertes oder mit Halogen, -CN, $-CONR^9R^{10}$, $-OR^9$, $-SR^9$ oder $-COOR^9$ substituiertes $C_1$-$C_{20}$-Alkyl$-(X)_p$ mit Ausnahme von $C_1$-$C_4$-Alkyl und Methoxy, $C_2$-$C_{18}$-Alkenyl$-(X)_p$, $C_2$-$C_{18}$-Alkinyl$-(X)_p$, $C_3$-$C_8$-Cycloalkyl$-(X)_p$, ($C_1$-$C_{12}$-Alkyl)-$C_3$-$C_8$-cycloalkyl$-(X)_p$, $C_3$-$C_8$-Cycloalkyl-$C_rH_{2r}-(X)_p$, ($C_1$-$C_{12}$-Alkyl)-$C_3$-$C_8$-cycloalkyl-$C_rH_{2r}-(X)_p$, Phenyl$-(X)_p$, ($C_1$-$C_{12}$-Alkyl)phenyl$-(X)_p$, Phenyl-$C_rH_{2r}-(X)_p$, ($C_1$-$C_{12}$-Alkyl)phenyl-$C_rH_{2r}-(X)_p$ darstellen,

r für 1 oder 2 und p für 0 oder 1 stehen und X -O-, -S-, -SO-oder $-SO_2-$ bedeutet,

oder $R^5$ bis $R^8$ unabhängig einen Substituenten aus der Gruppe -Br, $-CF_3$, -CN, $-Si(C_1$-$C_4$-$Alkyl)_3$, $-S-(C_mH_{2m}O)_n-R^{11}$ oder $-O-(C_mH_{2m}O)_n-R^{11}$ bedeuten,

oder je eines von $R^5$ bis $R^8$ für -F oder -Cl und mindestens ein weiteres von $R^5$ bis $R^8$ einen Substituenten der zuvor definierten Gruppen einschliesslich $C_1$-$C_4$-Alkyl und Methoxy bedeutet,

$R^9$ und $R^{10}$ unabhängig voneinander H, $C_1$-$C_{12}$-Alkyl, Phenyl oder $-(C_mH_{2m}O)_q-R^{11}$, oder $R^9$ und $R^{10}$ zusammen Tetramethylen, Pentamethylen, 3-Oxapentylen oder $-CH_2CH_2NR^9CH_2CH_2-$ bedeu-

ten,
$R^{11}$ für H oder $C_1$-$C_{12}$-Alkyl steht,
m für eine Zahl von 2 bis 4, n für eine Zahl von 2 bis 20 und q für eine Zahl von 1 bis 20 stehen, oder

b) $R^1$ bis $R^8$ unabhängig voneinander H oder einen der zuvor definierten Substituenten einschliesslich $C_1$-$C_4$-Alkyl und Methoxy darstellen, oder -COOR$^9$ oder -CONR$^9$R$^{10}$ oder je zwei benachbarte Reste von $R^1$ bis $R^8$ -CO-O-CO- oder -CO-NR$^9$-CO- bedeuten, und mindestens eines von $R^1$ bis $R^4$ sowie $R^5$ bis $R^8$ einen Substituenten bedeuten, mit Ausnahme von $R^2$, $R^3$, $R^6$ und $R^7$ gleich -F und Methyl, und wobei $R^9$ und $R^{10}$ die zuvor angegebenen Bedeutungen haben.

2. Verbindungen gemäss Anspruch 1, worin $R^1$, $R^4$, $R^5$ und $R^8$ für H stehen.

3. Verbindungen gemäss Anspruch 1, worin $R^6$ oder $R^7$, oder $R^6$ und $R^7$ einen Substituenten darstellen und $R^1$ bis $R^5$ und $R^8$ H bedeuten.

4. Verbindungen gemäss Anspruch 1, worin $R^6$ oder $R^6$ und $R^7$ sowie $R^2$ oder $R^2$ und $R^3$ einen Substituenten darstellen und $R^1$, $R^4$, $R^5$ und $R^8$ H bedeuten.

5. Verbindungen gemäss Anspruch 1, worin $R^1$ bis $R^4$ H sind,
und $R^5$ bis $R^8$ je für H stehen und mindestens einer der Reste $R^5$ bis $R^8$ unabhängig voneinander einen Substituenten aus der Gruppe unsubstituiertes oder mit -F, -Cl, -CN, -CONR$^9$R$^{10}$, -OR$^9$, -SR$^9$ oder -COOR$^9$ substituiertes $C_1$-$C_{18}$-Alkyl$-(X)_p$, $C_3$-$C_{12}$-Alkenyl$-(X)_p$, $C_3$-$C_{12}$-Alkinyl$-(X)_p$, $C_5$- oder $C_6$-Cycloalkyl$-(X)_p$, ($C_1$-$C_6$-Alkyl)-$C_5$- oder $C_6$-cycloalkyl$-(X)_p$, $C_5$- oder $C_6$-Cycloalkyl-$CH_2-(X)_p$, ($C_1$-$C_6$-Alkyl)-$C_5$- oder $C_6$-cycloalkyl-$CH_2-(X)_p$, Phenyl$-(X)_p$, ($C_1$-$C_6$-Alkyl)phenyl$-(X)_p$, Benzyl$-(X)_p$ oder ($C_1$-$C_6$-Alkyl)benzyl$-(X)_p$ darstellen;
X -O-, -S-, -SO- oder -$SO_2$- und p 0 oder 1 bedeuten;
oder $R^5$ bis $R^8$ unabhängig einen Substituenten aus der Gruppe -$CF_3$, -CN, -Si($C_1$- oder $C_2$-Alkyl)$_3$, -S$-(C_mH_{2m}$-O$)_n$ $R^{11}$, oder -O$-(C_mH_{2m}$-O$)_p$ $R^{11}$;
oder je eines von $R^5$ bis $R^8$ für -F oder -Cl und mindestens ein weiteres von $R^5$ bis $R^8$ einen Substituenten der zuvor definierten Gruppen einschliesslich $C_1$-$C_4$-Alkyl und Methoxy darstellen;
$R^9$ und $R^{10}$ unabhängig voneinander H, $C_1$-$C_6$-Alkyl, -$CH_2CH_2OH$, oder $R^9$ und $R^{10}$ zusammen Tetramethylen, Pentamethylen oder -$CH_2CH_2NR^9CH_2CH_2$- bedeuten; $R_{11}$ für H oder $C_1$-$C_4$-Alkyl steht; und m 2 oder 3 und n eine Zahl von 2 bis 12 darstellen.

6. Verbindungen gemäss den Ansprüchen 1 oder 5, worin p für 1 steht.

7. Verbindungen gemäss Anspruch 5, worin $R^1$ bis $R^5$ und $R^8$ H sind und $R^6$ ein Substituent und $R^7$ H oder $R^6$ und $R^7$ ein Substituent aus der Gruppe -Si($CH_3$)$_3$; -$CF_3$; -CN; mit -COOR$^9$ substituiertes $C_1$-$C_6$-Alkyl oder Benzyl; unsubstituiertes oder mit -OH substituiertes $C_1$-$C_{18}$-Alkoxy oder $C_1$-$C_{18}$-Alkylthio; unsubstituiertes oder mit -F, -Cl, -OH, $C_1$-$C_4$-Alkoxy, $C_1$-$C_4$-Alkylthio substituiertes Phenyl-X-, Benzyl-X-, $C_1$-$C_4$-Alkylphenyl-X- oder $C_1$-$C_4$-Alkylbenzyl-X- mit X gleich -O-, -S- oder -$SO_2$-; oder unsubstituiertes $C_3$-$C_{12}$-Alkenyloxy darstellen.

8. Verbindungen gemäss Anspruch 1, worin $R^1$, $R^4$, $R^5$ und $R^8$ H sind, und mindestens eines von $R^2$ und $R^3$ sowie von $R^6$ und $R^7$ einen Substituenten aus der Gruppe -COOR$^9$, -CONR$^9$R$^{10}$, unsubstituiertes oder mit -F, -Cl, -CN, -NR$^9$R$^{10}$, -OR$^9$, -SR$^9$ oder -COOR$^9$ substituiertes $C_1$-$C_{18}$-Alkyl$-(X)_p$, $C_3$-$C_{12}$-Alkenyl$-(X)_p$, $C_3$-$C_{12}$-Alkinyl$-(X)_p$, $C_5$- oder $C_6$-Cycloalkyl$-(X)_p$, ($C_1$-$C_6$-Alkyl)-$C_5$-oder $C_6$-cycloalkyl$-(X)_p$, $C_5$- oder $C_6$-Cycloalkyl-$CH_2-(X)_p$, ($C_1$-$C_6$-Alkyl)-$C_5$- oder $C_6$-cycloalkyl-$CH_2-(X)_p$, Phenyl$-(X)_p$, ($C_1$-$C_6$-Alkyl)phenyl$-(X)_p$, Benzyl$-(X)_p$ oder ($C_1$-$C_6$-Alkyl)benzyl$-(X)_p$ darstellen;
X -O-, -S-, -SO- oder -$SO_2$- und p 0 oder 1 bedeuten; oder aus der Gruppe -$CF_3$, -CN, -NR$^9$R$^{10}$, -Si($C_1$- oder $C_2$-Alkyl)$_3$, -S$-(C_mH_{2m}$-O$)-$ $R^{11}$, oder -O$-(C_mH_{2m}$-O$)-$ $R^{11}$; oder
$R^2$ und $R^3$ zusammen -CO-O-CO- oder -CO-NR$^9$-CO- sind und mindestens eines von $R^6$ und $R^7$ einen der zuvor definierten Substituenten darstellen oder gemeinsam -CO-O-CO- oder -CO-NR$^9$-CO- sind;
$R^9$ und $R^{10}$ unabhängig voneinander H, $C_1$-$C_6$-Alkyl, -$CH_2$-$CH_2OH$, oder $R^9$ und $R^{10}$ zusammen Tetramethylen, Pentamethylen oder -$CH_2CH_2NR^9CH_2CH_2$- bedeuten; $R_{11}$ für H oder $C_1$-$C_4$-Alkyl steht; und m 2 oder 3 und n eine Zahl von 2 bis 12 darstellen.

9. Verbindungen gemäss Anspruch 8, worin p für 1 steht.

10. Verbindungen gemäss Anspruch 8, worin der Substituent ausgewählt ist aus der Gruppe -COOR$^9$; -Si($CH_3$)$_3$; -$CF_3$; -CN; mit -COOR$^9$ substituiertes $C_1$-$C_6$-Alkyl oder Benzyl; unsubstituiertes oder mit -OH substituiertes $C_1$-$C_{18}$-Alkoxy oder $C_1$-$C_{18}$-Alkylthio; unsubstituiertes oder mit -F, -Cl, -OH, $C_1$-$C_4$-Alkoxy, $C_1$-$C_4$-Alkylthio substituiertes Phenyl-X-, Benzyl-X-, $C_1$-$C_4$-Alkylphenyl-X- oder $C_1$-$C_4$-Alkylbenzyl-X- mit X gleich -O-, -S- oder -$SO_2$-; oder unsubstituiertes $C_3$-$C_{12}$-Alkenyloxy.

11. Verbindungen gemäss Anspruch 8, worin der Substituent ausgewählt ist aus der Gruppe -$CF_3$, $C_1$-$C_{18}$-Alkoxy und -COOR$^9$.

12. Verfahren zur Herstellung von Verbindungen der Formel I gemäss Anspruch 1, dadurchgekennzeichnet, dass man eine Verbindung der Formel II

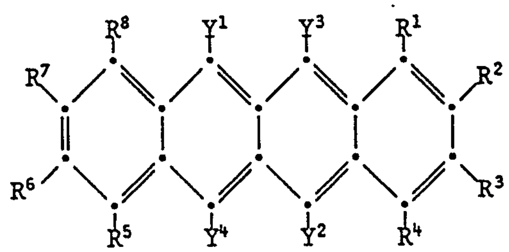

(II)

worin $R^1$ bis $R^8$ die in Anspruch 1 angegebenen Bedeutungen haben, $Y^1$ und $Y^2$ -Cl und $Y^3$ und $Y^4$ H bedeuten oder $Y^3$ und $Y^4$ -Cl und $Y^1$ und $Y^2$ H bedeuten, mit Schwefel, Selen, einem Alkalimetallsulfid oder -selenid umsetzt.